# EUROPÄISCHE PATENTSCHRIFT

(11) **EP 3 064 192 B1**
(45) Veröffentlichungstag und Bekanntmachung des Hinweises auf die Patenterteilung: **07.11.2018**
(21) Anmeldenummer: 15158108.9
(22) Anmeldetag: 06.03.2015
(51) Int. Cl.: A61K 6/00, A61K 6/083

(54) **Dentalmaterialien auf der Basis von Hybridmonomeren**
Dental materials on the basis of hybrid monomers
Matériaux dentaires à base de monomères hybrides

(43) Veröffentlichungstag der Anmeldung: 07.09.2016
(73) Patentinhaber: Ivoclar Vivadent AG, 9494 Schaan (LI)
(72) Erfinder: Moszner, Norbert, 9495 Triesen (LI); Angermann, Jörg, 7320 Sargans (CH); Fischer, Urs-Karl, 9320 Arbon (CH); Hauner, Martina, 6800 Feldkirch (AT)
(74) Vertreter: Uexküll & Stolberg

(56) Entgegenhaltungen:
- US-A- 5 741 543
- US-A1- 2014 120 326
- US-B1- 6 197 906
- NIE J ET AL: "Synthesis and characterization of N-isopropyl, N-methacryloxyethyl methacrylamide as a possible dental resin", BIOMATERIALS, ELSEVIER SCIENCE PUBLISHERS BV., BARKING, GB, Bd. 22, Nr. 6, 15. März 2001 (2001-03-15), Seiten 535-540, XP004313858, ISSN: 0142-9612, DOI: 10.1016/S0142-9612(00)00209-X

## Beschreibung

Die vorliegende Erfindung betrifft thermisch- und/oder lichthärtende Dentalwerkstoffe mit sehr guten mechanischen Eigenschaften, die sich insbesondere als dentale Füllungskomposite und als Werkstoff für Inlay, Onlays, Kronen, Brücken oder Verblendmaterialien eignen.

Die polymerisierbare organische Matrix von dentalen Füllungskompositen und Adhäsiven basiert meist auf einer Mischung von Dimethacrylaten (vgl. A. Peutzfeldt, Resin composites in dentistry: the monomer systems, Eur. J. Oral. Sci. 105 (1997) 97-116; J. W. Nicolson, H. M. Anstice, The chemistry of modern dental filling materials, J. Chem. Ed. 76 (1999) 1497-1501; J. W. Stansburry, Curing dental resins and composites by photopolymerization, J. Esthet. Dent., 12 (2000) 300-308; N. Moszner, T. Hirt, New Polymer-Chemical Developments in Clinical Dental Polymer Materials: Enamel-Dentin Adhesives and Restorative Composites, J. Polym. Sci. Part A: Polym. Chem. 50 (2012) 4369-4402). Beispiele für häufig eingesetzte Dimethacrylate sind die hochviskosen Vernetzer 2,2-Bis[4-(2-hydroxy-3-methacryloyloxypropyl)phenyl]propan (Bis-GMA) und 1,6-Bis-[2-methacryloyloxyethoxycarbonylamino]-2,4,4-trimethylhexan
(UDMA) oder die als Verdünnermonomere genutzten niedrigviskosen Dimethacrylate, wie z.B. Bis(methacryloyloxymethyl)-tricyclo[5.2.1.]decan (TCDMA), Decandiol-1,10-dimethacrylat (D₃MA) und Triethylenglycoldimethacrylat (TEGDMA). Für wässrige Adhäsive eignen sich als Vernetzer auch Bisacrylamide, wie z.B. N,N'-Diethyl-1,3-bis(acrylamido)-propan, die sich unter wässrig-sauren Bedingungen im Vergleich zu den Dimethacrylaten durch eine deutlich höhere Hydrolysestabilität auszeichnen (vgl. N. Moszner, U. Salz, J. Zimmermann, Dent. Mater. 21 (2005) 895-910).

Monomere, die zwei unterschiedliche polymerisationsfähige Gruppen enthalten, bezeichnet man als Hybridmonomere. Die EP 0 792 882 A1 offenbart Dentalmaterialien, die polymerisierbare Hybridmonomere enthalten, die neben Norbornenyl- oder Norbornadienyl-Gruppen auch (Meth)acrylat-, Vinyl-, Allyl-, Allylether-, Vinylether-, Epoxy- oder Styrylgruppen aufweisen. Diese Monomere können nach verschiedenen Mechanismen polymerisiert werden, so dass eine mehrstufige Polymerisation, die Kombination von verschiedenen Polymerisationsmechanismen oder die Synthese von polymerisierbaren Polymeren möglich ist.

Chan et al., Austr. J. Chem. 51 (1998) 31-35, beschreiben die Synthese von Hybridmonomeren, die sowohl (Meth)acrylamid- als auch (Meth)acrylatgruppen enthalten. Diese Monomere sollen sich als Vernetzer eignen, wenn Löslichkeit sowohl in organischen als auch wässrigen Systemen erforderlich ist oder wenn die Anwesenheit einer leicht hydrolysierbaren Gruppe zum kontrollierten Abbau des Polymernetzwerks erwünscht ist.

Die EP 1 182 033 A1 offenbart Materialien zur Bildaufzeichnung, die ein polymeres Bindemittel mit unterschiedlichen radikalisch polymerisierbaren Gruppen enthalten können.

JP 2001270856 A offenbart ein Verfahren zur Herstellung von N-(Meth)acryloyloxyethylacrylamiden durch Umesterung. Das Verfahren soll die gewünschten Produkte in hoher Ausbeute und Reinheit ergeben.

Die WO 2010/019832 offenbart Amid-verlängerte Maleinsäureimide, die sich zur Verstärkung von thermisch härtenden Adhäsiven eignen sollen, ohne deren Hitzestabilität zu beeinträchtigen. Unter "Amid-verlängert" werden Verbindungen verstanden, die mindestens eine Amidgruppe in nicht terminaler Position des Moleküls enthalten.

Die WO 96/24644 A1 betrifft druckempfindliche Adhäsive, die sich zur Beschichtung von Gegenständen eignen sollen. Die Adhäsive können mehrfach ungesättigte Monomere enthalten, deren ungesättigte Gruppen unterschiedliche Reaktivitäten aufweisen. Durch die unterschiedlichen Reaktivitäten soll ein vorzeitiges Gelieren verhindert werden.

Die WO 98/01419 offenbart Gele für die Gelelektrophorese, die asymmetrische Vernetzer enthalten. Es handelt sich hierbei um Moleküle mit zwei unterschiedlich reaktiven Vinylgruppen. Durch die Reaktion der reaktiveren Gruppe sollen zunächst lineare Polymerketten gebildet werden, die anschließend über die zweite Gruppe miteinander vernetzt werden, so dass Gele mit einer zur Trennung von Biomolekülen vorteilhaften mikroporösen Struktur entstehen.

Jun Nie et al. ("Synthesis and characterization of N-isopropyl,N-methacryloxyethyl methacrylamide as a possible dental resin", Biomaterials, Bd. 22, Nr. 6, 15. März 2001, S. 535-540,) beschreibt einen polymerisierbaren Dentalwerkstoff, der N-Isopropyl,N-methacryloxyethyl-methacrylamid enthält.

Der Erfindung liegt die Aufgabe zugrunde, dentale Werkstoffe bereitzustellen, die sich im Vergleich zu auf Dimethacrylaten basierenden Dentalmaterialien durch verbesserte mechanische Eigenschaften auszeichnen.

Die Aufgabe wird erfindungsgemäß durch Dentalwerkstoffe gelöst, die mindestens eine radikalisch polymerisierbare Verbindung gemäß der allgemeinen Formel I enthalten: 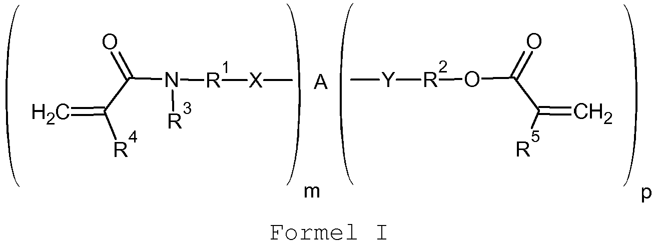 in der die Variablen die folgenden Bedeutungen haben:
- A: ein aliphatischer linearer oder verzweigter C₁-C₁₅-Kohlenwasserstoffrest, der durch ein oder mehrere O unterbrochen sein kann;
- X, Y: unabhängig voneinander jeweils entfällt oder -NH-CO-O-, wobei X und Y nicht gleichzeitig entfallen,
- R¹,R²: unabhängig voneinander jeweils entfällt, ein aliphatischer linearer oder verzweigter C₁-C₁₀-Alkylen-Rest, der durch ein oder mehrere O unterbrochen sein kann, wobei A, R¹ und R² zusammen mindestens 3 C-Atome enthalten,
- R³: ein aliphatischer linearer C₁-C₃-Alkyl-Rest,
- R⁴,R⁵: unabhängig voneinander jeweils Wasserstoff, Methyl oder Ethyl, und
- m,p: unabhängig voneinander jeweils eine ganze Zahl von 1 bis 3.

In den Verbindungen der Formel I ist A ein m+p-wertiger Rest, an den jeweils m Acrylamidgruppen (linker Klammerausdruck) und p Acrylatgruppen (rechter Klammerausdruck) gebunden sind. Es handelt sich um Hybridmonomere, die 2 bis 6, vorzugweise 2 bis 3 radikalisch polymerisierbare Gruppen aufweisen.

Die Gruppierung -R¹-X-A-Y-R²- bildet einen Spacer, der die polymerisierbare(n) Acrylamidgruppe(n) mit der(den) polymerisierbaren Acrylatgruppe(n) verbindet. Verbindungen mit einem linearen Spacer sind bevorzugt, d.h. die mindestens 3 C-Atome der Reste A, R¹ und R² sind vorzugsweise linear angeordnet. Ebenso sind R¹, R² und A vorzugsweise lineare Gruppen. In den Verbindungen der Formel (I) ist mindestens einer der Reste X und Y -NH-CO-O-.

Die Formel I erstreckt sich nur auf solche Verbindungen, die mit der chemischen Valenzlehre vereinbar sind. Beispielsweise kann die Summe von m und p dann, wenn A ein C₁-Rest ist, maximal 4 sein. Der Hinweis, dass ein Rest durch einen oder mehrere O-Atome, S-Atome etc. unterbrochen ist, ist so zu verstehen, dass diese Gruppen jeweils in die Kohlenstoffkette des Restes eingeschoben werden. Diese Gruppen sind damit beidseitig durch C-Atome begrenzt und können nicht endständig sein. C₁-Reste können nicht unterbrochen sein.

Es wurde überraschend gefunden, dass Verbindungen der Formel I bei der radikalischen Polymerisation Materialien ergeben, die sich gegenüber Materialien mit herkömmlichen dentalen Vernetzern durch deutlich verbesserte mechanische Eigenschaften auszeichnen.

Bevorzugt sind Verbindungen, in denen die Variablen der Formel I die folgenden Bedeutungen haben:
- A: ein aliphatischer verzweigter, vorzugsweise linearer C₁-C₈-Kohlenwasserstoffrest, der durch ein oder mehrere O unterbrochen sein kann,
- X,: -NH-CO-O- oder vorzugsweise entfällt,
- Y: entfällt oder vorzugsweise -NH-CO-O-, wobei X und Y nicht gleichzeitig entfallen,
- R¹: entfällt,
- R²: entfällt oder ein aliphatischer linearer C₁-C₂-Alkylen-Rest,
- R³: Methyl oder Ethyl,
- R⁴,R⁵: unabhängig voneinander Wasserstoff oder Methyl, und
- m,p: unabhängig voneinander jeweils 1 oder 2.

Die Verbindungen der allgemeinen Formel I sind teilweise aus dem Stand der Technik bekannt und lassen sich durch einfache Syntheseverfahren herstellen. Beispielsweise lassen sich (m+p)-funktionelle α,ω-Aminoalkohole mit α,β-ungesättigten Carbonsäurechloriden in Gegenwart einer Hilfsbase zu di- oder höherfunktionalisierten Hybridmonomeren der allgemeinen Formel I umsetzen, allgemeines Beispiel:

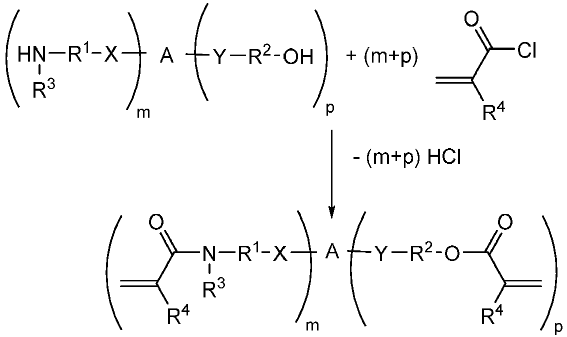

Ein konkretes Beispiel ist:

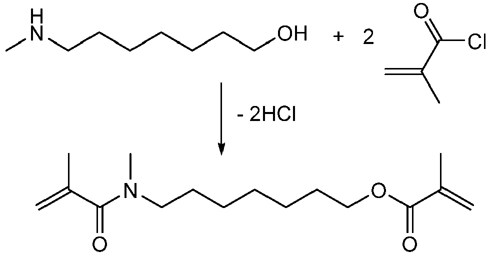

Aufgrund der größeren Nucleophilie von Aminogruppen im Vergleich zu OH-Gruppen kann die Einführung der unterschiedlichen polymerisationsfähigen (Meth)acrylamid und (Meth)acrylatGruppen auch schrittweise erfolgen. Beispielsweise wird der (m+p)-funktionelle α,ω-Aminoalkohol zunächst bei tiefer Temperatur mit dem α,β-ungesättigten Carbonsäurechlorid CH₂=CHR⁴-COCl umgesetzt und danach mit dem α,β-ungesättigten Carbonsäurechlorid CH₂=CHR⁵-COCl bei Raumtemperatur acyliert:

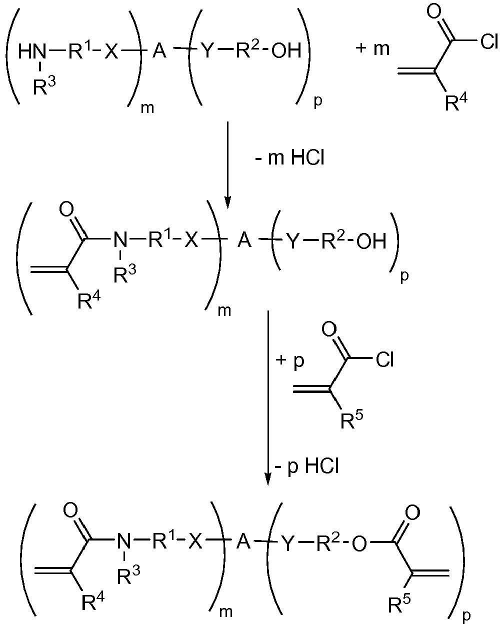

Ein konkretes Beispiel ist:

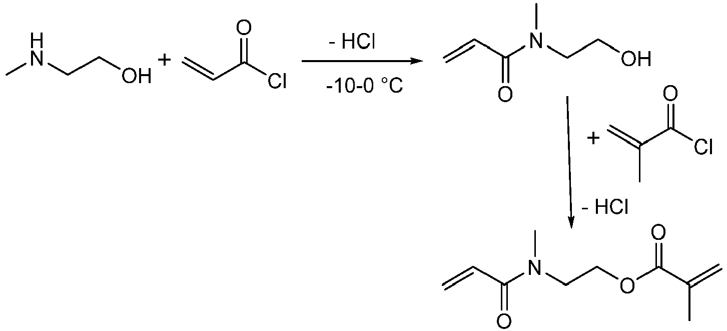

Bevorzugte Beispiele für die erfindungsgemäßen di- oder höherfunktionellen Hybridmonomeren der allgemeinen Formel I sind:

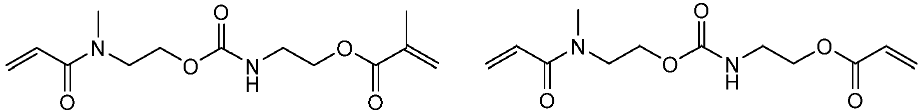

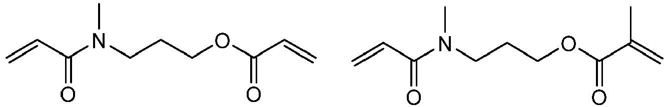

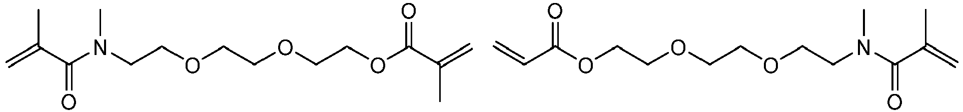

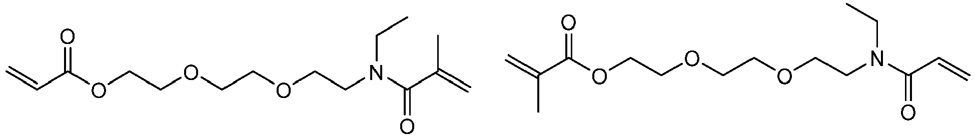

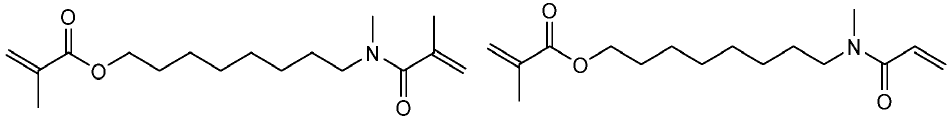

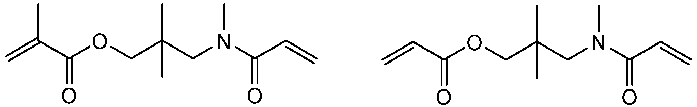

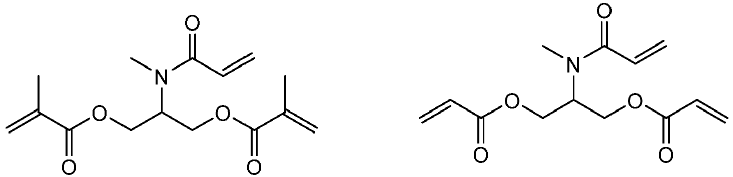

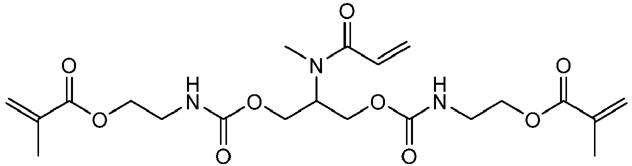

Die erfindungsgemäßen Dentalmaterialien enthalten neben den Monomeren der allgemeinen Formel I vorzugsweise zusätzlich weitere radikalisch polymerisierbare Monomere, insbesondere mono- und/oder polyfunktionelle (Meth)acrylsäurederivate. Besonders bevorzugt sind Werkstoffe, die als radikalisch polymerisierbares Monomer mindestens ein multifunktionelles (Meth)acrylat oder eine Mischung von mono- und multifunktionellen (Meth)acrylaten enthalten. Unter monofunktionellen (Meth)acrylaten werden Verbindungen mit einer, unter polyfunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten. Materialien, die als radikalisch polymerisierbares Monomer mono- und multifunktionelle (Meth)acrylaten enthalten, eignen sich besonders als Dentalwerkstoffe. In allen Fällen sind Methacrylate als Co-Monomere bevorzugt. Es wurde gefunden, dass die Monomere der Formel I und insbesondere die bevorzugten Verbindungen der Formel I mit den hier genannten Co-Monomeren gut verträglich sind und homogene Mischungen bilden, die bei der Polymerisation Materialien mit sehr guten mechanischen Eigenschaften ergeben. Bevorzugt sind Verbindungen der Formel I, die unter Standardbedingungen flüssig sind.

Bevorzugte mono- oder polyfunktionelle Methacrylate sind Methyl-, Ethyl-, Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), TMX-UDMA (ein Additionsprodukt aus einer Mischung von HEMA und Hydroxypropylmethacrylat (HPMA) mit α,α,α',α'-Tetramethyl-m-xylylendiisocyanat (TMXDI)), Bis(methacryloyl-oxymethyl)tricyclo[5.2.1.]decan (TCDMA), ethoxy- oder propoxyliertes Bisphenol-A-di(meth)acrylat, wie z.B. das Bisphenol-A-dimethacrylat 2-[4-(3-Methacryloyloxyethoxyethyl)-phenyl]-2-[4-(3-methacryloyloxyethyl)phenyl]-propan) (SR-348c) mit 3 Ethoxygruppen oder 2,2-Bis[4-(2-(meth)acryloxypropoxy)-phenyl]propan, Di-, Tri- oder Tetraethylenglycoldi(meth)-acrylat, Trimethylolpropantri(meth)acrylat, Pentaerythrit-tetra(meth)acrylat, sowie Glycerindi(meth)acrylat, 1,4-Butan-dioldi(meth)acrylat, 1,10-Decandioldi(meth)acrylat oder 1,12-Dodecandioldi(meth)acrylat oder Glycerintrimethacrylat (GTMA).

Weiter bevorzugt sind N-mono- oder -disubstituierte Acrylamide, wie z.B. N-Ethylacrylamid, N,N-Dimethylacrylamid, N-(2-Hydroxyethyl)acrylamid oder N-Methyl-N-(2-hydroxyethyl)acrylamid, bzw. N-monosubstituierte Methacrylamide, wie z.B. N-Ethylmethacrylamid oder N-(2-Hydroxyethyl)methacrylamid sowie N-Vinylpyrrolidon. Diese Monomere zeichnen sich durch eine geringe Viskosität und eine hohe Hydrolysestabilität aus und eignen sich besonders als Verdünnermonomere.

Ebenfalls bevorzugt sind vernetzende Pyrrolidone, wie z.B. 1,6-Bis(3-vinyl-2-pyrrolidonyl)-hexan oder kommerziell zugängliche Bisacrylamide, wie Methylen- oder Ethylenbisacrylamid, bzw. Bis(meth)acrylamide, wie z.B. N,N'-Diethyl-1,3-bis(acryl-amido)-propan, 1,3-Bis(methacrylamido)-propan, 1,4-Bis(acryl-amido)-butan oder 1,4-Bis(acryloyl)-piperazin, die durch Umsetzung aus den entsprechenden Diaminen mit (Meth)acrylsäurechlorid synthetisiert werden können. Diese Monomere zeichnen sich durch eine hohe Hydrolysestabilität aus und eignen sich besonders als Vernetzermonomere.

Besonders bevorzugte Comonomere sind: CMP-1E, Bis-GMA, UDMA, TMX-UDMA, TCDMA, ethoxy- oder propoxyliertes Bisphenol-A-dimethacrylat, SR-348c, Triethylenglycoldimethacrylat, Glycerindimethacrylat, 1,10-Decandioldimethacrylat oder Glycerintrimethacrylat (GTMA) sowie N,N'-Diethyl-1,3-bis(acrylamido)-propan.

Alternativ oder zusätzlich können die erfindungsgemäßen Dentalwerkstoffe neben den oben genannten Co-Monomeren ein oder mehrere säuregruppenhaltige radikalisch polymerisierbare Monomere (Haftmonomere) als Zusatzmonomere enthalten. Diese verleihen den Werkstoffen selbsthaftende und/oder selbstätzende Eigenschaften.

Bevorzugte säuregruppenhaltige Monomere sind polymerisationsfähige Carbonsäuren, Phosphonsäuren, Phosphorsäureester, und Sulfonsäuren.

Bevorzugte Carbonsäuren sind Maleinsäure, Acrylsäure, Methacrylsäure, 2-(Hydroxymethyl)acrylsäure, 4-(Meth)acryloyloxy-ethyltrimellitsäure, 10-Methacryloyloxydecylmalonsäure, N-(2-Hydroxy-3-methacryloyloxypropyl)-N-phenylglycin bzw. 4-Vinylbenzoesäure.

Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure oder 2-[4-(Di-hydroxyphosphoryl)-2-oxa-butyl]-acrylsäureethyl- und -2,4,6-trimethylphenylester.

Bevorzugte acide polymerisationsfähige Phosphorsäureester sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat.

Bevorzugte polymerisationsfähige Sulfonsäuren sind Vinylsulfonsäure, 4-Vinylphenylsulfonsäure oder 3-(Methacrylamido)-propylsulfonsäure.

Besonders bevorzugte Säuremonomere sind 4-(Meth)acryloyloxy-ethyltrimellitsäureanhydrid, 10-Methacryloyloxydecylmalonsäure, 2-Methacryloyloxyethylphosphonsäure, 2-Methacrylamidoethylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- oder -2,4,6-trimethylphenylester, 2-Methacryloyloxypropylmono- oder -dihydrogenphosphat, 2-Methacryloyloxyethylmono- oder -dihydrogenphosphat, 2-Methacryloyloxyethylphenylhydrogenphosphat, 10-Methacryloyloxydecyl-dihydrogenphosphat, 6-(Methacrylamido)hexyldihydrogenphosphat und 1,3-Bis-(N-acryloyl-N-propyl-amino)-propan-2-yl-dihydrogenphosphat.

Die erfindungsgemäßen Dentalwerkstoffe enthalten vorzugsweise auch einen Initiator für die radikalische Polymerisation.

Zur Initiierung der radikalischen Photopolymerisation sind Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil bevorzugt. Besonders bevorzugt werden Campherchinon und 2,2-Dimethoxy-2-phenyl-acetophenon und besonders bevorzugt α-Diketone in Kombination mit Aminen als Reduktionsmittel, wie z.B. 4-(Dimethylamino)benzoesäureethylester, N,N-Dimethylaminoethylmethacrylat, N,N-Dimethyl-sym.-xylidin oder Triethanolamin, verwendet. Besonders geeignet sind auch Norrish-Typ-I-Photoinitiatoren, vor allem Acyl- oder Bisacylphosphinoxide, Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium. Dabei lassen sich vorteilhaft auch Mischungen der verschiedenen Photoinitiatoren einsetzen, wie z.B. Dibenzoyldiethylgermanium in Kombination mit Campherchinon und 4-Dimethylaminobenzoesäureethylester.

Als Initiatoren für eine bei Raumtemperatur durchgeführte Polymerisation werden vorzugsweise Redox-Initiatorkombinationen, wie z.B. Kombinationen von Benzoylperoxid mit N,N-Dimethyl-sym.-xylidin oder N,N-Dimethyl-p-toluidin, verwendet. Darüber hinaus sind auch Redoxsysteme bestehend aus Peroxiden bzw. Hydroperoxiden und solchen Reduktionsmitteln, wie z.B. Ascorbinsäure, Barbiturate, Thioharnstoffe oder Sulfinsäuren, besonders geeignet.

Weiterhin enthalten die erfindungsgemäßen Dentalwerkstoffe vorzugsweise auch mindestens einen organischen oder besonders bevorzugt anorganischen partikulären Füllstoff. Bevorzugt sind Füllstoffe auf der Basis von Oxiden, wie SiO₂, ZrO₂ und TiO₂ bzw. Mischoxiden aus SiO₂, ZrO₂, ZnO und/oder TiO₂, nanopartikuläre oder mikrofeine Füllstoffe, wie pyrogene Kieselsäure oder Fällungskieselsäure (gewichtsmittlere Partikelgröße von 10-1000 nm) sowie Minifüllstoffe, wie Quarz-, Glaskeramik- oder röntgenopake Glaspulver von z.B. Barium- oder Strontiumaluminiumsilikatgläsern (gewichtsmittlere Partikelgröße von 0,2-10 µm). Weitere bevorzugte Füllstoffe sind röntgenopake Füllstoffe, wie Ytterbiumtrifluorid oder nanopartikuläres Tantal(V)-oxid bzw. Bariumsulfat oder Mischoxide von SiO₂ mit Ytterbium(III)-oxid oder Tantal(V)-oxid (gewichtsmittlere Partikelgröße von 10-1000 nm).

Zur Verbesserung des Verbundes zwischen den Füllstoffpartikeln und der vernetzten Polymerisationsmatrix können SiO₂-basierende Füllstoffe mit methacrylat-funktionalisierten Silanen, wie z.B. 3-Methacryloyloxypropyltrimethoxysilan, oberflächenmodifiziert werden. Zur Oberflächenmodifizierung von nichtsilikatischen Füllstoffen, z.B. von ZrO₂ oder TiO₂, können auch funktionalisierte saure Phosphate, wie z.B. 10-Methacryloyloxydihydrogenphospaphat eingesetzt werden.

Die erfindungsgemäßen Dentalwerkstoffe können in Abhängigkeit vom gewünschten Einsatzzweck vorzugsweise auch ein Lösungsmittel enthalten, insbesondere Wasser, Ethanol oder eine Mischung davon.

Gegebenenfalls können die erfindungsgemäß eingesetzten Zusammensetzungen außerdem weitere Additive enthalten, vor allem Stabilisatoren, wie z.B. Polymerisationsstabilisatoren, Aromastoffe, Farbmittel, mikrobiozide Wirkstoffe, fluoridionenabgebende Additive, optische Aufheller, Fluoreszensmittel, Weichmacher, und/oder UV-Absorber.

Dabei sind erfindungsgemäß solche Dentalmaterialien bevorzugt, die die folgenden Komponenten enthalten:
a) 0,1 bis 50 Gew.-%, bevorzugt 1 bis 40 Gew.-% und besonders bevorzugt 2 bis 30 Gew.-% mindestens einer Verbindung der allgemeinen Formel I,
b) 0,01 bis 10 Gew.-%, besonders bevorzugt 0,1 bis 3,0 Gew.-% mindestens eines Initiators, und ggf.
c) 0 bis 80 Gew.-%, bevorzugt 0 bis 60 Gew.-% und besonders bevorzugt 5 bis 50 Gew.-% andere(s) Monomer(en), und ggf.
d) 0 bis 80 Gew.-% Füllstoff(e), und ggf.
e) 0 bis 70 Gew.-% Lösungsmittel.

Dentalwerkstoffe zur Verwendung als Zement oder Füllungskomposit weisen bevorzugt folgende Zusammensetzung auf:
a) 0,1 bis 40 Gew.-%, bevorzugt 1 bis 30 Gew.-% und besonders bevorzugt 5 bis 30 Gew.-% mindestens einer Verbindung der allgemeinen Formel I,
b) 0,01 bis 10 Gew.-%, besonders bevorzugt 0,1 bis 3,0 Gew.-% mindestens eines Initiators,
c) 0 bis 50 Gew.-%, bevorzugt 0 bis 40 Gew.-% und besonders bevorzugt 5 bis 40 Gew.-% andere(s) Monomer(e),
d) 10 bis 80 Gew.-%, bevorzugt 20 bis 80 Gew.-%, besonders bevorzugt 30 bis 80 Gew.-% Füllstoff(e).

Dentalwerkstoffe zur Verwendung als Adhäsive oder Beschichtungsmaterial weisen bevorzugt folgende Zusammensetzung auf:
a) 0,1 bis 50 Gew.-%, bevorzugt 1 bis 40 Gew.-% und besonders bevorzugt 2 bis 30 Gew.-% mindestens einer Verbindung der allgemeinen Formel I,
b) 0,01 bis 10 Gew.-%, besonders bevorzugt 0,1 bis 3,0 Gew.-% mindestens eines Initiators,
c) 0 bis 80 Gew.-%, bevorzugt 5 bis 60 Gew.-% und besonders bevorzugt 5 bis 50 Gew.-% andere(s) Monomer(e),
d) 0 bis 20 Gew.-% Füllstoff(e),
e) 0 bis 70 Gew.-%, bevorzugt 0 bis 60 Gew.-% und besonders bevorzugt 0 bis 50 Gew.-% Lösungsmitteln, insbesondere Wasser und/oder Ethanol.

Dentalwerkstoffe zur Herstellung von Prothesen oder künstlichen Zähnen weisen bevorzugt folgende Zusammensetzung auf:
a) 0,1 bis 50 Gew.-%, bevorzugt 1 bis 40 Gew.-% und besonders bevorzugt 2 bis 30 Gew.-% mindestens einer Verbindung der allgemeinen Formel I,
b) 0,01 bis 10 Gew.-%, besonders bevorzugt 0,1 bis 3,0 Gew.-% mindestens eines Initiators, und
c) 0 bis 80 Gew.-%, bevorzugt 0 bis 60 Gew.-% und besonders bevorzugt 5 bis 50 Gew.-% andere(s) Monomer(e), und
d) 0 bis 40 Gew.-% Füllstoff(e).

Dentalwerkstoffe zur Herstellung von Inlays, Onlays, Kronen oder Brücken weisen bevorzugt folgende Zusammensetzung auf:
a) 0,1 bis 50 Gew.-%, bevorzugt 1 bis 40 Gew.-% und besonders bevorzugt 2 bis 30 Gew.-% mindestens einer Verbindung der allgemeinen Formel I,
b) 0,01 bis 10 Gew.-%, besonders bevorzugt 0,1 bis 3,0 Gew.-% mindestens eines Initiators, und ggf.
c) 0 bis 60 Gew.-%, bevorzugt 0 bis 50 Gew.-% und besonders bevorzugt 5 bis 50 Gew.-% andere(s) Monomer(e), und ggf.
d) 10 bis 80 Gew.-%, bevorzugt 15 bis 80 Gew.-% und besonders bevorzugt 20 bis 80 Gew.-% Füllstoff(e).

Wenn nicht anders angegeben beziehen sich alle Mengenangaben auf die Gesamtmasse der Materialien. Die einzelnen Mengenbereiche können separat gewählt werden.

Besonders bevorzugt sind solche Werkstoffe, welche aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind.

Die erfindungsgemäßen Werkstoffe eignen sich besonders als Dentalwerkstoffe, insbesondere als dentale Adhäsive, Zemente, Füllungskomposite und Verblendmaterialien sowie als Materialien zur Herstellung von Prothesen, künstlichen Zähnen, Inlays, Onlays, Kronen und Brücken. Sie zeichnen sich gegenüber auf Dimethacrylaten basierende Materialien durch deutlich verbesserte mechanische Eigenschaften (Biegefestigkeit und E-Modul) aus.

Die Dentalwerkstoffe eignen sich primär zur intraoralen Anwendung durch den Zahnarzt zur Restauration geschädigter Zähne (klinische Materialien), z.B. als dentale Zemente, Füllungskomposite und Verblendmaterialien. Sie können aber auch extraoral eingesetzt werden, beispielsweise bei der Herstellung oder Reparatur von Dentalrestaurationen, wie Prothesen, künstlichen Zähnen, Inlays, Onlays, Kronen und Brücken (technische Materialien).

Die Erfindung wird im Folgenden anhand von Ausführungsbeispielen näher erläutert.

### Ausführungsbeispiele

### Beispiel 1:

### Synthese von 2-{[(2-(N-Methylacrylamido)-ethoxy)-carbonyl]-amino}-ethylmethacrylat (MAMMA)

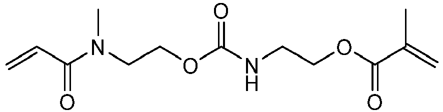

Zu einer Lösung von 15,2 mg Bismut(III)-neodecanoat und 8,5 mg BHT in 21,2 g (0,164 mol) *N*-(2-Hydroxyethyl)-*N*-methyl-acrylamid wurden innerhalb von 30 min 25,4 g (0,164 mol) 2-Isocyanatoethylmethacrylat zugetropft. Das Reaktionsgemisch erwärmte sich dabei bis auf ca. 56 °C und wurde anschließend bei 50°C Badtemperatur gerührt. Der Verlauf der Reaktion wurde durch IR-Spektroskopie und Titration verfolgt. Nach 11 h bei 50°C wurden 44,8 g (96% der Theorie) MAMMA als klare, farblose Flüssigkeit mit einer Reinheit von 96.95% (HPLC) erhalten. *n_{D}²⁰* = 1,4990; *η* (23°C) = 1,84 Pa·s ¹H-NMR (400 MHz, CDCl₃): 2 Rotamere (ca. 1:1), *δ* (ppm) = 1.94 (s, 3H, CH_{3*,Methacryl*}), 3.03 und 3.13 (2 s, je 1.5 H, NCH₃), 3.48 (q, *J* = 5.6 Hz, 2H, HN-CH₂), 3.63 und 3.68 (2 t, je *J* = 5.7 Hz, je 1H, H₃CN-CH₂), 4.19-4.26 (m, 4H, OCH₂), 5.40 (s, 1H, NH), 5.60 und 6.12 (2 s, je 1H, C=CH₂), 5.65-5.72, 6.29-6.34 und 6.55-6.62 (3 m, je 1H, HC=CH₂).
¹³C-NMR (100 MHz, CDCl₃): 2 Rotamere (ca. 1:1), *δ* (ppm) = 18.3 (CH_{3, *Methacryl*}), 34.1 und 36.5 (NCH₃), 40.1 und 40.2 (HN-CH₂), 47.4 und 48.8 (NCH₂), 61.8, 62.3, 63.5 und 63.6 (OCH₂), 126.0 und 126.1 (C=CH₂), 127.3 und 127.5 (HC=CH₂), 128.0 und 128.2 (HC=CH₂), 135.9 (C=CH₂), 156.0 und 156.3 (C=O*_{Urethan}*), 166.6 und 166.8 (C=O*_{Amid}*), 167.2 (C=O*_{Ester}*).
IR (Diamant-ATR): *v* (cm⁻¹) = 3299 (m, NH), 2955 (m, CH₂, CH₃), 1712 (vs, C=O*_{Ester}*), 1645 (s, C=O*_{Amid}*), 1610 (s, C=C), 1532 (s, NH), 1451 (s, CH₂, CH₃), 1251 (s, C-N), 1153 (vs, COC), 948 (s, =CH) .

### Beispiel 2:

### Polymerisationsharze auf der Basis von MAMMA aus Beispiel 1

Es wurden Monomermischungen (Harze) A bis D (Anteile in Gew.-%) durch Mischen von UDMA, Campherchinon (CQ), 4-(Dimethylamino)benzoesäureethylester (EDMAB) mit den hochviskosen Monomeren TMX-UDMA (ein Additionsprodukt aus einer Mischung von HEMA und Hydroxypropylmethacrylat (HPMA) mit α,α,α',α'-Tetramethyl-m-xylylendiisocyanat) oder TCM-UDMA (Umsetzungsprodukt von 1 Mol 4,8-Bis(hydroxymethyl)tricyclo[5.2.1.0(2,6)]decan mit 2 Mol 2-Isocyanatoethylmethacrylat) und den Verdünnermonomeren MAMMA aus Beispiel 1 oder Glycerintrimethacrylat (GTMA) hergestellt (Tabelle 1):

**Tabelle 1: Radikalisch polymerisierbare Monomermischungen**

| **Komponenten** | **Harz A** | **Harz B** | **Harz C^{*)}** | **Harz D^{*)}** | **Harz E^{**)}** |
|---|---|---|---|---|---|
| **UDMA** | 52,58 | 52,58 | 52,58 | 52,58 | 52,58 |
| **TMX-UDMA** | 14,88 | - | 14,88 | - | - |
| **TCM-UDMA** | - | 14,88 | - | 14,88 | - |
| **BisGMA** | - | - | - | - | 14,88 |
| **MAMMA** | 31,74 | 31,74 | - | - | - |
| **GTMA** | - | - | 31,74 | 31,74 | - |
| **TEGDMA** | - | - | - | - | 31,74 |
| **CQ** | 0,30 | 0,30 | 0,30 | 0,30 | 0,30 |
| **EDMAB** | 0,50 | 0,50 | 0,50 | 0,50 | 0,50 |

| | | | | | |
|---|---|---|---|---|---|
| ^{*)} Vergleichsbeispiel ^{**)} kommerzielles Harz, Vergleichsbeispiel | | | | | |

Von den Harzen wurden Prüfkörper präpariert (Abmessung 2 x 2 x 25 mm), die 2 x 3 Minuten mit einer dentalen Lichtquelle (Spectramat®, Ivoclar Vivadent AG) bestrahlt und damit ausgehärtet wurden. Nach der ISO-Norm 4049 (Dentistry - Polymer-based filling, restorative and luting materials) erfolgte die Bestimmung der Biegefestigkeit und des Biege-E-Moduls (Tabelle 2) .

**Tabelle 2: Mechanische Eigenschaften der gehärteten Harze**

| **Eigenschaft** | **Harz A** | **Harz B** | **Harz C^{*)}** | **Harz D^{*)}** | **Harz E^{*)}** |
|---|---|---|---|---|---|
| **Biegefestigkeit (MPa)** | 105,1±2,3 | 109,0±2,2 | 59,9+5,8 | 56,5+8,9 | 63,3±1,4 |
| **Biege-E-Modul (GPa)** | 2,45±0,09 | 2,49±0,23 | 1,49±0,06 | 2,01±0,10 | 1,89±0,13 |

| | | | | | |
|---|---|---|---|---|---|
| ^{*)} Vergleichsbeispiel | | | | | |

Die Ergebnisse belegen die sehr guten mechanischen Eigenschaften von Polymerisaten mit dem erfindungsgemäßen Monomer MAMMA als Verdünnermonomer, auch im Vergleich zum trifunktionellen Verdünnermonomer GTMA. Ein übliches Dentalharz analoger Zusammensetzung (Harz E), bei dem einerseits das hochviskoses Dimethacrylat Bis-GMA und andererseits das Verdünnermonomer TEGDMA eingesetzt wurde, ergab Polymerisate mit deutlich schlechteren mechanischen Eigenschaften.

### Beispiel 3:

### Kompositzement auf der Basis von MAMMA aus Beispiel 1

Mit einem Dreiwalzenstuhl wurden folgende Kompositzementpasten (Anteile in Gew.-%) hergestellt (Tabelle 3):

**Tabelle3: Zusammensetzungen von Dentalzementen**

| **Komponente** | **Zement A** | **Zement B^{*)}** |
|---|---|---|
| **UDMA** | 35,67 | 35,67 |
| **MAMMA** | 8,83 | - |
| **D₃MA** | - | 8,83 |
| **CQ** | 0,27 | 0,27 |
| **EDMAB** | 0,26 | 0,26 |
| **Ox-50¹⁾** | 37, 83 | 37,83 |
| **YbF₃²⁾** | 17,14 | 17,14 |

| | | |
|---|---|---|
| ^{*)} Vergleichsbeispiel ¹⁾ silanisierte pyrogene Kieselsäure (Fa. Degussa) mit einer Primärpartikelgröße von 40 nm ²⁾ Ytterbiumtrifluorid (Fa. Auer Remy) mit einer mittleren Partikelgröße von 0,2 µm | | |

Von den Zementpasten wurden Prüfkörper präpariert (Abmessung 2 x 2 x 25 mm), die 2 x 3 Minuten mit einer dentalen Lichtquelle (Spectramat®, Ivoclar Vivadent AG) ausgehärtet wurden. Nach der ISO-Norm ISO-4049 (Dentistry - Polymer-based filling, restorative and luting materials) erfolgte die Bestimmung der Biegefestigkeit und des Biege-E-Moduls (Tabelle 4). Die Ergebnisse belegen die sehr guten mechanischen Eigenschaften des Zementes mit dem erfindungsgemäßen Monomer MAMMA als Verdünnermonomer im Vergleich zum Zement mit dem üblichen dentalen Verdünnermonomer D₃MA.

**Tabelle 4: Mechanische Eigenschaften der gehärteten Harze**

| **Eigenschaft** | **Zement A** | **Zement B^{*)}** |
|---|---|---|
| **Biegefestigkeit (MPa)** | 114,9±6,9 | 89,0±10,2 |
| **Biege-E-Modul (GPa)** | 6,04±0,21 | 4,38±0,29 |

| | | |
|---|---|---|
| ^{*)} Vergleichsbeispiel | | |

### Beispiel 4:

### Füllungskomposit auf der Basis von MAMMA aus Beispiel 1

In einem Kneter der Firma Linden wurden die Komposite A und B hergestellt (Tabelle 5):

**Tabelle 5: Zusammensetzung von Füllungskompositen**

| **Komponente** | **Komposit A** | **Komposit B^{*)}** | **Komposit C^{**)}** |
|---|---|---|---|
| **Bis-GMA** | 11,85 | 11,85 | 11,85 |
| **UDMA** | 4,33 | 4,33 | 4,33 |
| **MAMMA** | 5,66 | - | - |
| **TEGDMA** | - | 5,66 | - |
| **Bisacrylamid⁵⁾** | - | - | 5,66 |
| **CQ** | 0,09 | 0,09 | 0,09 |
| **EDMAB** | 0,11 | 0,11 | 0,11 |
| **ox-50¹⁾** | 10,87 | 10,87 | 10,87 |
| **YbF₃²⁾** | 14,62 | 14,62 | 14,62 |
| **Sphärosil³⁾** | 8,60 | 8,60 | 8,60 |
| **Glasfüller⁴⁾** | 43,87 | 43,87 | 43,87 |

| | | | |
|---|---|---|---|
| ^{*)} Vergleichsbeispiel ^{**)} kommerzielles Füllungskomposit, Vergleichsbeispiel ¹⁾ silanisierte pyrogene Kieselsäure (Fa. Degussa) mit einer Primärpartikelgröße von 40 nm ²⁾ Ytterbiumtrifluorid (Fa. Auer Remy) mit einer mittleren Partikelgröße von 0,2 µm ³⁾ silanisiertes SiO₂-ZrO₂-Mischoxid (Fa. Tokuyama Soda) mit einer mittleren Partikelgröße von 1,2 µm ⁴⁾ Silanisiertes Al-Borosilikatglas (Fa. Schott) mit einer mittleren Partikelgröße von 0,7 µm ⁵⁾ N,N-Diethyl-1,3-bis(acrylamidopropan) | | | |

Von den Kompositpasten wurden Prüfkörper präpariert (Abmessung 2 x 2 x 25 mm), die 2 x 3 Minuten mit einer dentalen Lichtquelle (Spectramat®, Ivoclar Vivadent AG) ausgehärtet wurden. Nach der ISO-Norm ISO-4049 (Dentistry - Polymer-based filling, restorative and luting materials) erfolgte die Bestimmung der Biegefestigkeit und des Biege-E-Moduls (Tabelle 6).

**Tabelle 6: Mechanische Eigenschaften gehärteter Komposite**

| **Eigenschaft** | **Komposit A** | **Komposit B^{*)}** | **Komposit C^{*)}** |
|---|---|---|---|
| **Biegefestigkeit (MPa)** | 140,9±7,6 | 116,5±12,2 | 120,2±14,9 |
| **Biege-E-Modul (GPa)** | 8,47±0,40 | 6,66±0,43 | 6,97±0,64 |

| | | | |
|---|---|---|---|
| ^{*)} Vergleichsbeispiel | | | |

Die Ergebnisse belegen die sehr guten mechanischen Eigenschaften des Komposits mit dem erfindungsgemäßen Monomer MAMMA als Verdünnermonomer im Vergleich zu einem Komposit mit dem üblichen dentalen Verdünnermonomer TEGDMA. Ein übliches Komposit analoger Zusammensetzung, bei dem als Verdünnermonomer das in Dentalwerkstoffen häufig genutzte Bisacrylamid N,N'-Diethyl-1,3-bis(acrylamido)-propan eingesetzt wurde, ergab Komposite mit schlechteren mechanischen Eigenschaften. Komposit A eignet sich besonders als dentales Füllungsmaterial.

## Patentansprüche

1. Dentalwerkstoff, der mindestens eine Verbindung der Formel I enthält, 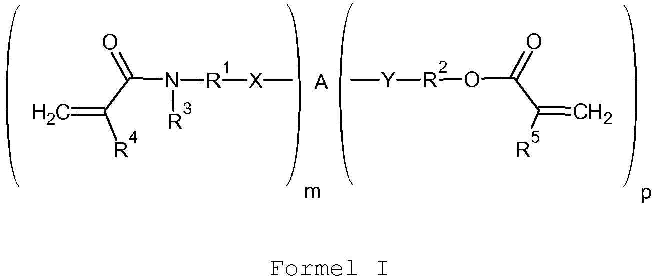 in der die Variablen die folgenden Bedeutungen haben:
A ein aliphatischer linearer oder verzweigter C₁-C₁₅-Kohlenwasserstoffrest, der durch ein oder mehrere O unterbrochen sein kann;
X,Y unabhängig voneinander jeweils entfällt oder -NH-CO-O-, wobei X und Y nicht gleichzeitig entfallen,
R¹,R² unabhängig voneinander jeweils entfällt, ein aliphatischer linearer oder verzweigter C₁-C₁₀-Alkylen-Rest, der durch ein oder mehrere O unterbrochen sein kann, wobei A, R¹ und R² zusammen mindestens 3 C-Atome enthalten,
R³ ein aliphatischer linearer C₁-C₃-Alkyl-Rest,
R⁴,R⁵ unabhängig voneinander jeweils Wasserstoff, Methyl oder Ethyl, und
m,p unabhängig voneinander jeweils eine ganze Zahl von 1 bis 3.

2. Dentalwerkstoff nach Anspruch 1, wobei die Variablen der Formel I die folgenden Bedeutungen haben:
A ein aliphatischer linearer oder verzweigter C₁-C₈-Kohlenwasserstoffrest, der durch ein oder mehrere O unterbrochen sein kann,
X, entfällt oder -NH-CO-O-,
Y entfällt oder -NH-CO-O-, wobei X und Y nicht gleichzeitig entfallen,
R¹ entfällt,
R² entfällt, ein aliphatischer linearer C₁-C₂-Alkylen-Rest,
R³ Methyl oder Ethyl,
R⁴,R⁵ unabhängig voneinander Wasserstoff oder Methyl, und
m, p unabhängig voneinander jeweils 1 oder 2.

3. Werkstoff nach einem der Ansprüche 1 bis 2, der zusätzlich mindestens ein radikalisch polymerisierbares Monomer und vorzugsweise mindestens einen Initiator für die radikalische Polymerisation enthält.

4. Werkstoff nach Anspruch 3, der mindestens ein multifunktionelles (Meth)acrylat oder eine Mischung von mono- und multifunktionellen (Meth)acrylaten enthält.

5. Werkstoff nach einem der Ansprüche 1 bis 4, der mindestens einen Füllstoff enthält.

6. Werkstoff nach einem der Ansprüche 1 bis 5, der
a) 0,1 bis 50 Gew.-%, bevorzugt 1 bis 40 Gew.-% und besonders bevorzugt 2 bis 30 Gew.-% mindestens einer Verbindung der allgemeinen Formel I,
b) 0,01 bis 10 Gew.-%, besonders bevorzugt 0,1 bis 3,0 Gew.-% mindestens eines Initiators,
c) 0 bis 80 Gew.-%, bevorzugt 0 bis 60 Gew.-% und besonders bevorzugt 5 bis 50 Gew.-% andere(s) Monomer(e),
d) 0 bis 80 Gew.-% Füllstoff(e) und
e) 0 bis 70 Gew.-% Lösungsmittel enthält,
jeweils bezogen auf die Gesamtmasse des Werkstoffs.

7. Werkstoff nach Anspruch 6 zur Verwendung als dentaler Zement oder dentales Füllungskomposit, der
a) 0,1 bis 40 Gew.-%, bevorzugt 1 bis 30 Gew.-% und besonders bevorzugt 5 bis 30 Gew.-% mindestens einer Verbindung der allgemeinen Formel I,
b) 0,01 bis 10 Gew.-%, besonders bevorzugt 0,1 bis 3,0 Gew.-% mindestens eines Initiators,
c) 0 bis 50 Gew.-%, bevorzugt 0 bis 40 Gew.-% und besonders bevorzugt 5 bis 40 Gew.-% andere(s) Monomer(e),
d) 10 bis 80 Gew.-%, bevorzugt 20 bis 80 Gew.-%, besonders bevorzugt 30 bis 80 Gew.-% Füllstoff(e) enthält.

8. Werkstoff nach Anspruch 6 zur Verwendung als dentales Adhäsiv oder Beschichtungsmaterial, der
a) 0,1 bis 50 Gew.-%, bevorzugt 1 bis 40 Gew.-% und besonders bevorzugt 2 bis 30 Gew.-% mindestens einer Verbindung der allgemeinen Formel I,
b) 0,01 bis 10 Gew.-%, besonders bevorzugt 0,1 bis 3,0 Gew.-% mindestens eines Initiators,
c) 0 bis 80 Gew.-%, bevorzugt 5 bis 60 Gew.-% und besonders bevorzugt 5 bis 50 Gew.-% andere(s) Monomer(e),
d) 0 bis 20 Gew.-% Füllstoff(e),
e) 0 bis 70 Gew.-%, bevorzugt 0 bis 60 Gew.-% und besonders bevorzugt 0 bis 50 Gew.-% Lösungsmittel, insbesondere Wasser und/oder Ethanol enthält.

9. Werkstoff nach Anspruch 6 zur Herstellung von dentalen Prothesen oder künstlichen Zähnen, der
a) 0,1 bis 50 Gew.-%, bevorzugt 1 bis 40 Gew.-% und besonders bevorzugt 2 bis 30 Gew.-% mindestens einer Verbindung der allgemeinen Formel I,
b) 0,01 bis 10 Gew.-%, besonders bevorzugt 0,1 bis 3,0 Gew.-% mindestens eines Initiators, und
c) 0 bis 80 Gew.-%, bevorzugt 0 bis 60 Gew.-% und besonders bevorzugt 5 bis 50 Gew.-% andere(s) Monomer(e),
d) 0 bis 40 Gew.-% Füllstoff(e) enthält.

10. Werkstoff nach Anspruch 6 zur Herstellung von Inlays, Onlays, Kronen oder Brücken, der
a) 0,1 bis 50 Gew.-%, bevorzugt 1 bis 40 Gew.-% und besonders bevorzugt 2 bis 30 Gew.-% mindestens einer Verbindung der allgemeinen Formel I,
b) 0,01 bis 10 Gew.-%, besonders bevorzugt 0,1 bis 3,0 Gew.-% mindestens eines Initiators, und ggf.
c) 0 bis 60 Gew.-%, bevorzugt 0 bis 50 Gew.-% und besonders bevorzugt 5 bis 50 Gew.-% andere(s) Monomer(e), und ggf.
d) 10 bis 80 Gew.-%, bevorzugt 15 bis 80 Gew.-% und besonders bevorzugt 20 bis 80 Gew.-% Füllstoff(e) enthält.

11. Werkstoff nach einem der Ansprüche 1 bis 8 zur intraoralen Verwendung zur Restauration geschädigter Zähne.

12. Werkstoff nach Anspruch 11 zur Verwendung als dentaler Zement, dentaler Füllungskomposit, dentales Adhäsiv oder Verblendmaterial.

13. Verwendung eines Werkstoffs nach einem der Ansprüche 1 bis 10 als Material zur extraoralen Herstellung oder Reparatur von Dentalrestaurationen.

14. Verwendung nach Anspruch 13 zur Herstellung von künstlichen Zähnen, Prothesen, Inlays, Onlays, Kronen oder Brücken.

## Claims

1. Dental material which comprises at least one compound of Formula I 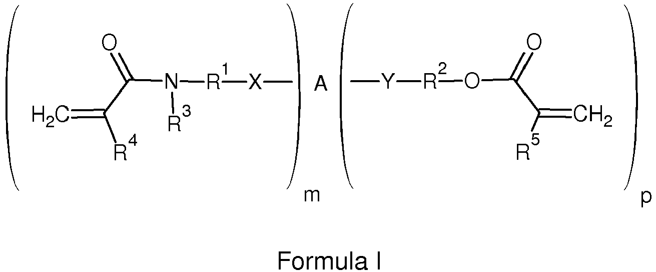 in which the variables have the following meanings:
A is an aliphatic linear or branched C₁-C₁₅ hydrocarbon group that may be interrupted by one or more O,
X, Y independently of each other in each case are absent or are -NH-CO-O-, wherein X and Y are not simultaneously absent
R¹, R² independently of each other is absent, an aliphatic linear or branched C₁-C₁₀ alkylene group that may be interrupted by one or more O, wherein A, R¹ and R² together contain at least 3 C atoms,
R³ is an aliphatic linear C₁-C₃ alkyl group,
R⁴, R⁵ independently of each other are each hydrogen, methyl or ethyl, and
m, p independently of each other are each an integer from 1 to 3.

2. Dental material according to claim 1, wherein the variables of Formula I have the following meanings:
A is an aliphatic linear or branched C₁-C₈ hydrocarbon group that may be interrupted by one or more O,
X is absent or -NH-CO-O-,
Y is absent or -NH-CO-O-, wherein X and Y are not simultaneously absent,
R¹ is absent,
R² is absent, an aliphatic linear or branched C₁-C₂ alkylene group,
R³ is methyl or ethyl,
R⁴, R⁵ independently of each other are hydrogen or methyl, and
m, p independently of each other are each 1 or 2.

3. Material according to one of claims 1 to 2 which additionally comprises at least one radically polymerisable monomer and preferably at least one initiator for the radical polymerisation.

4. Material according to claim 3 which comprises at least one multifunctional (meth)acrylate or a mixture of mono- and multifunctional (meth)acrylates.

5. Material according to one of claims 1 to 4 which comprises at least one filler.

6. Material according to one of claims 1 to 5 which comprises
a) 0.1 to 50 wt %, preferably 1 to 40 wt % and particularly preferably 2 to 30 wt % of at least one compound of the general formula I,
b) 0.01 to 10 wt %, particularly preferably 0.1 to 3.0 wt % of at least one initiator,
c) 0 to 80 wt %, preferably 0 to 60 wt % and particularly preferably 5 to 50 wt % of other monomer(s),
d) 0 to 80 wt % of filler(s) and
e) 0 to 70 wt % solvent,
in each case relative to the total mass of the material.

7. Material according to claim 6 for use as a dental cement or dental filling composite which comprises
a) 0.1 to 40 wt %, preferably 1 to 30 wt % and particularly preferably 5 to 30 wt % of at least one compound of the general formula I,
b) 0.01 to 10 wt %, particularly preferably 0.1 to 3.0 wt % of at least one initiator,
c) 0 to 50 wt %, preferably 0 to 40 wt % and particularly preferably 5 to 40 wt % of other monomer(s),
d) 10 to 80 wt %, preferably 20 to 80 wt %, particularly preferably 30 to 80 wt % filler(s).

8. Material according to claim 6 for use as a dental adhesive or coating material which comprises
a) 0.1 to 50 wt %, preferably 1 to 40 wt % and particularly preferably 2 to 30 wt % of at least one compound of the general formula I,
b) 0.01 to 10 wt %, particularly preferably 0.1 to 3.0 wt % of at least one initiator,
c) 0 to 80 wt %, preferably 5 to 60 wt % and particularly preferably 5 to 50 wt % of other monomer(s),
d) 0 to 20 wt % filler(s),
e) 0 to 70 wt %, preferably 0 to 60 wt % and particularly preferably 0 to 50 wt % of solvents, in particular water and/or ethanol.

9. Material according to claim 6 for the manufacture of dental prostheses or artificial teeth which comprises
a) 0.1 to 50 wt %, preferably 1 to 40 wt % and particularly preferably 2 to 30 wt % of at least one compound of the general formula I,
b) 0.01 to 10 wt %, particularly preferably 0.1 to 3.0 wt % of at least one initiator, and
c) 0 to 80 wt %, preferably 0 to 60 wt % and particularly preferably 5 to 50 wt % of other monomer(s),
d) 0 to 40 wt % filler(s).

10. Material according to claim 6 for the manufacture of inlays, onlays, crowns or bridges which comprises
a) 0.1 to 50 wt %, preferably 1 to 40 wt % and particularly preferably 2 to 30 wt % of at least one compound of the general formula I,
b) 0.01 to 10 wt %, particularly preferably 0.1 to 3.0 wt % of at least one initiator, and optionally
c) 0 to 60 wt %, preferably 0 to 50 wt % and particularly preferably 5 to 50 wt % of other monomer(s), and optionally
d) 10 to 80 wt %, preferably 15 to 80 wt % and particularly preferably 20 to 80 wt % of filler(s).

11. Material according to one of claims 1 to 8 for intraoral use to restore damaged teeth.

12. Material according to claim 11 for use as a dental cement, dental filling composite, dental adhesive or veneering material.

13. Use of a material according to one of claims 1 to 10 as a material for the extraoral manufacture or repair of dental restorations.

14. Use according to claim 13 for the manufacture of artificial teeth, prostheses, inlays, onlays, crowns or bridges.

## Revendications

1. Matériau dentaire, qui contient au moins un composé de formule I 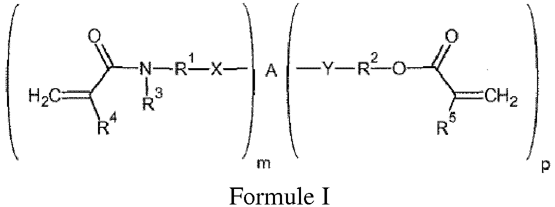 dans laquelle les variables ont les significations suivantes :
A représente un radical hydrocarboné aliphatique en C₁-C₁₅ linéaire ou ramifié, qui peut être interrompu par un ou plusieurs atome(s) d'oxygène;
X, Y chacun, indépendamment l'un de l'autre, est absent ou est un groupe -NH-CO-O-, X et Y n'étant pas absents simultanément,
R¹, R² chacun, indépendamment l'un de l'autre, est absent ou représente un radical alkylène aliphatique en C₁-C₁₀ linéaire ou ramifié, qui peut être interrompu par un ou plusieurs atome(s) d'oxygène, A, R¹ et R² contenant ensemble au moins 3 atomes de carbone,
R³ représente un radical alkyle aliphatique en C₁-C₃ linéaire,
R⁴, R⁵ représentent chacun, indépendamment l'un de l'autre, un atome d'hydrogène, un groupe méthyle ou éthyle, et
m, p représentent chacun, indépendamment l'un de l'autre, un nombre entier valant de 1 à 3.

2. Matériau dentaire selon la revendication 1, dans lequel les variables de la formule I ont les significations suivantes :
A représente un radical hydrocarboné aliphatique en C₁-C₈ linéaire ou ramifié, qui peut être interrompu par un ou plusieurs atome(s) d'oxygène ;
X est absent ou est un groupe -NH-CO-O-,
Y est absent ou est un groupe -NH-CO-O-, X et Y n'étant pas absents simultanément,
R¹ est absent,
R² est absent ou est un radical alkylène aliphatique en C₁-C₂ linéaire,
R³ représente un radical méthyle ou éthyle,
R⁴, R⁵ représentent chacun, indépendamment l'un de l'autre, un atome d'hydrogène ou un groupe méthyle, et
m, p valent chacun, indépendamment l'un de l'autre, 1 ou 2.

3. Matériau selon l'une quelconque des revendications 1 et 2, qui contient en outre au moins un monomère polymérisable par voie radicalaire et de préférence au moins un amorceur pour la polymérisation radicalaire.

4. Matériau selon la revendication 3, qui contient au moins un (méth)acrylate multifonctionnel ou un mélange de (méth)acrylates monofonctionnels et de (méth)acrylates multifonctionnels.

5. Matériau selon l'une quelconque des revendications 1 à 4, qui contient au moins une charge.

6. Matériau selon l'une quelconque des revendications 1 à 5, qui contient
a) 0,1 à 50 % en poids, de préférence 1 à 40 % en poids et de façon particulièrement préférée 2 à 30 % en poids d'au moins un composé de formule générale I,
b) 0,01 à 10 % en poids, de façon particulièrement préférée 0,1 à 3,0 % en poids d'au moins un amorceur,
c) 0 à 80 % en poids, de préférence 0 à 60 % en poids et de façon particulièrement préférée 5 à 50 % en poids d'un autre/d'autres monomère(s),
d) 0 à 80 % en poids de charge(s) et
e) 0 à 70 % en poids de solvant,
chaque fois par rapport à la masse totale du matériau.

7. Matériau selon la revendication 6, destiné à l'utilisation en tant que ciment dentaire ou composite d'obturation dentaire, qui contient
a) 0,1 à 40 % en poids, de préférence 1 à 30 % en poids et de façon particulièrement préférée 5 à 30 % en poids d'au moins un composé de formule générale I,
b) 0,01 à 10 % en poids, de façon particulièrement préférée 0,1 à 3,0 % en poids d'au moins un amorceur,
c) 0 à 50 % en poids, de préférence 0 à 40 % en poids et de façon particulièrement préférée 5 à 40 % en poids d'un autre/d'autres monomère(s),
d) 10 à 80 % en poids, de préférence 20 à 80 % en poids, de façon particulièrement préférée 30 à 80 % en poids de charge(s).

8. Matériau selon la revendication 6, destiné à l'utilisation en tant que matériau de revêtement ou adhésif dentaire, qui contient
a) 0,1 à 50 % en poids, de préférence 1 à 40 % en poids et de façon particulièrement préférée 2 à 30 % en poids d'au moins un composé de formule générale I,
b) 0,01 à 10 % en poids, de façon particulièrement préférée 0,1 à 3,0 % en poids d'au moins un amorceur,
c) 0 à 80 % en poids, de préférence 5 à 60 % en poids et de façon particulièrement préférée 5 à 50 % en poids d'un autre/d'autres monomère(s),
d) 0 à 20 % en poids de charge(s) et
e) 0 à 70 % en poids, de préférence 0 à 60 % en poids et de façon particulièrement préférée 0 à 50 % en poids de solvant, en particulier d'eau et/ou d'éthanol.

9. Matériau selon la revendication 6, destiné à la fabrication de prothèses dentaires ou de dents artificielles, qui contient
a) 0,1 à 50 % en poids, de préférence 1 à 40 % en poids et de façon particulièrement préférée 2 à 30 % en poids d'au moins un composé de formule générale I,
b) 0,01 à 10 % en poids, de façon particulièrement préférée 0,1 à 3,0 % en poids d'au moins un amorceur, et
c) 0 à 80 % en poids, de préférence 0 à 60 % en poids et de façon particulièrement préférée 5 à 50 % en poids d'un autre/d'autres monomère(s),
d) 0 à 40 % en poids de charge(s).

10. Matériau selon la revendication 6, destiné à la production d'inlays, d'onlays, de couronnes ou de bridges, qui contient
a) 0,1 à 50 % en poids, de préférence 1 à 40 % en poids et de façon particulièrement préférée 2 à 30 % en poids d'au moins un composé de formule générale I,
b) 0,01 à 10 % en poids, de façon particulièrement préférée 0,1 à 3,0 % en poids d'au moins un amorceur, et éventuellement
c) 0 à 60 % en poids, de préférence 0 à 50 % en poids et de façon particulièrement préférée 5 à 50 % en poids d'un autre/d'autres monomère(s), et éventuellement
d) 10 à 80 % en poids, de préférence 15 à 80 % en poids et de façon particulièrement préférée 20 à 80 % en poids de charge(s).

11. Matériau selon l'une quelconque des revendications 1 à 8, destiné à l'utilisation intra-orale pour la restauration de dents abîmées.

12. Matériau dentaire selon la revendication 11, destiné à l'utilisation en tant que ciment dentaire, composite d'obturation dentaire, adhésif dentaire ou matériau de placage.

13. Utilisation d'un matériau selon l'une quelconque des revendications 1 à 10 en tant que matériau pour la production extra-orale ou la réparation de restaurations dentaires.

14. Utilisation selon la revendication 13 pour la fabrication de dents artificielles, de prothèses, d'inlays, d'onlays, de couronnes ou de bridges.
